# FASCICULE DE BREVET EUROPEEN

(11) **EP 0 706 789 B1**
(45) Date de publication et mention de la délivrance du brevet: **15.05.2002**
(21) Numéro de dépôt: 95401985.7
(22) Date de dépôt: 31.08.1995
(51) Int. Cl.: A61K 7/48

(54) **Composition cosmétique comprenant des huiles siliconées**
Silikoneölen enthaltende kosmetische Zusammensetzung
Cosmetic composition containing silicone oils

(30) Priorité: 10.10.1994 FR 9412071
(43) Date de publication de la demande: 17.04.1996
(73) Titulaire: L'OREAL, 75008 Paris (FR)
(72) Inventeur: Bara, Isabelle, F-75014 Paris (FR); Terren, Nadia, F-94550 Chevilly Larue (FR); Michelet, Jacques, F-91160 Champlan (FR)
(74) Mandataire: Dodin, Catherine

(56) Documents cités:
- EP-A- 0 610 014
- EP-A- 0 613 679
- US-A- 4 780 145
- US-A- 4 917 891
- CHEMICAL ABSTRACTS, vol. 115, no. 16, 21 Octobre 1991 Columbus, Ohio, US; abstract no. 166398, N. WATANABE 'Water-in-oil cosmetic emulsions containing cyclic silicones' & JP-A-03 095 107 (KANEBO LTD.) 19 Avril 1991

## Description

La présente invention a trait à une composition cosmétique pouvant se présenter sous forme d'une émulsion huile-dans-eau ou eau-dans-huile, ou encore sous forme d'une pâte ou d'une poudre, comprenant des composés siliconés, et entre autre des huiles siliconées.

On connaît des compositions cosmétiques de maquillage, telles que des fonds de teint, se présentant sous forme d'une émulsion eau-dans-huile dans laquelle la phase huile, ou phase grasse, est constituée principalement de corps gras siliconés.
Après l'application d'une telle composition, l'eau s'évapore et les composés siliconés restent au contact de la peau, procurant un maquillage qui peut être résistant à l'eau. L'application de telles compositions présente toutefois l'inconvénient de laisser à la peau un aspect huileux et de procurer au toucher un effet gras. Le résultat obtenu après application n'est pas réellement naturel, le maquillage brille ou devient brillant.

Le document JP03095107 décrit des compositions comprenant soit une silicone cyclique tétramère, soit une silicone cyclique pentamère, soit une silicone cyclique hexamère, soit un mélange de silicones cycliques tétramère et pentamère. Les documents EP-A-0 613 679 et US 4, 780, 145 décrivent des compositions cosmétiques comprenant soit l'octaméthylcyclotétrasiloxane, soit le décaméthylcyclopentasiloxane. Le document EP-A-0 610 014 décrit des compositions comprenant un mélange d'octaméthylcyclotétrasiloxane et de décaméthylcyclopentasiloxane. Le document US 4, 917, 891 décrit des compositions comprenant soit le décaméthylcyclopentasiloxane soit l'hexaméthyldisiloxane.

La présente invention a pour but de proposer une composition cosmétique comprenant des pigments qui procure un maquillage agréable à réaliser, et qui reste non brillant au cours du temps, tout en évitant la sensation de gras au toucher.

Un objet de la présente invention est donc une composition selon la revendication 1.

Un autre objet de l'invention est l'utilisation selon la revendication 13.

Ainsi, lorsque l'on utilise la composition selon l'invention, les huiles siliconées volatiles s'évaporent au contact de la peau et permettent l'obtention d'un maquillage qui ne brille pas, et qui reste mat au cours du temps. De plus, le maquillage obtenu n'est pas huileux au toucher.

Un avantage de la composition selon l'invention est d'être aisée à appliquer, en s'étalant facilement et uniformément.
Un autre avantage est de présenter une texture légère et fluide, douce au toucher.
Un autre avantage est de permettre l'obtention d'un maquillage coloré naturel, de bonne couvrance et de bonne tenue.

La composition selon l'invention peut se présenter sous forme d'une émulsion huile-dans-eau, eau-dans-huile ou multiple, sous forme d'un stick ou bâton, sous forme d'une pâte anhydre ou non, ou encore sous forme d'une poudre compactée ou coulée.

La composition selon l'invention comprend donc dans une phase grasse, au moins l'huile cyclopentadiméthylsiloxane et l'huile cyclohexadiméthylsiloxane.
On a en effet constaté que, dans les compositions ainsi obtenues, la cyclopentadiméthylsiloxane contribue à assurer un maquillage aisé, en apportant du glissant à la composition et en en facilitant l'étalement sur la peau. Cette huile s'évapore ensuite rapidement après l'application. La cyclohexadiméthylsiloxane, dont l'évaporation est plus lente, apporte du confort au maquillage et permet de maintenir sa souplesse à la peau en évitant les tiraillements et une sensation de sec. De préférence, la composition comprend un mélange de cyclopentadiméthylsiloxane en une quantité de 2-18% en poids par rapport au poids total de la composition, et de cyclohexadiméthylsiloxane en une quantité de 2-18% en poids.

La composition peut éventuellement comprendre d'autres huiles volatiles. Par huile volatile, on entend dans la présente description, toute huile susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 50-100°C.
On emploie de préférence des huiles présentant des degrés de volatilité différents, de manière à conserver à la composition finale des qualités cosmétiques adéquates. On peut en particulier citer les huiles de silicones cycliques ou linéaires, telles que la cyclotétradiméthylsiloxane ou le X2-1731 de Dow Corning, et/ou les huiles organiques notamment de paraffine telles que les ISOPARs.
De préférence, la composition selon l'invention comprend 4-20% en poids d'huiles volatiles par rapport au poids total de la composition.

La composition peut comprendre, en plus des huiles volatiles, des constituants usuellement utilisés dans le domaine cosmétique.
Parmi ceux-ci, on peut citer les corps gras siliconés tels que les huiles de silicone non volatiles, les gommes de silicone, les cires de silicone, ainsi que les corps gras non siliconés tels que les huiles ou les cires végétales, minérales, organiques et/ou synthétiques.

Les huiles de silicone non volatiles utilisables dans la composition selon l'invention peuvent être des huiles de faible viscosité telles que les polysiloxanes linéaires, dont le degré de polymérisation est de préférence de 3 à 2000 environ.
L'huile peut être présente à raison de 0-15% en poids dans la composition finale, de préférence 10-13%. On peut citer, par exemple :
. les polydiméthylsiloxanes (PDMS) de viscosité inférieure à 100 mPa.s et de préférence inférieure à 10 mPa.s,
. les alkyldiméthicones répondant à la formule: 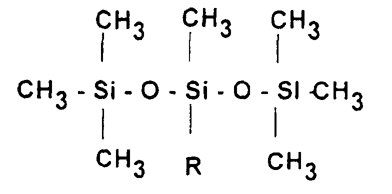 dans laquelle R représente le radical CₙH₂ₙ₊₁, avec n = 1 8.
. les polyphénylméthylsiloxanes.
On peut citer, par exemple, l'huile Silbione 70-047V de Rhône Poulenc, l'huile 200 de Dow Corning, l'huile polycétylméthylsiloxane de Goldschmidt.

Les gommes de silicone utilisables dans la composition selon l'invention peuvent être des polysiloxanes de masse moléculaire élevée, de l'ordre de 200 000 à 1 000 000.
Elles peuvent être utilisées seules ou en mélange avec un solvant tel qu'une huile polydiméthylsiloxane ou polyphénylsiloxane.
La gomme peut être présente à raison de 0-1% en poids de matière active dans la composition finale, de préférence à raison de 0,1-0,5%.
La gomme de silicone peut répondre à la formule : 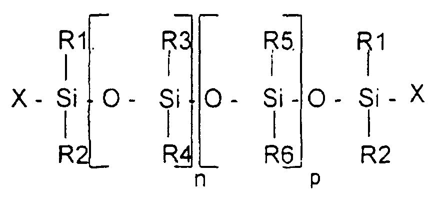 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre des valeurs de 0 à 5000, de préférence de 0 à 3000.
Comme gomme de silicone utilisable selon l'invention, on peut citer celles pour lesquelles :
. les substituants R1 à R6 et X représentent un groupement méthyle, p = 0 et n = 2700, comme celle vendue sous la dénomination SE30 par la société General Electric,
. les substituants R1 à R6 et X représentent un groupement méthyle, p = 0 et n = 2300, comme celle vendue sous la dénomination AK 500000 par la société Waker,
. les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
. les substituants R1, R2, R5, R6 et X représentent un groupement méthyle, les substituants R3 et R4 représentent un groupement aryle tel que le poids moléculaire du composé soit de 600 000, comme celle vendue sous la dénomination 761 par la société Rhône-Poulenc.

Les cires de silicone utilisable dans la composition selon l'invention peuvent être des polysiloxanes linéaires substitués. On peut citer, par exemple, les cires de silicone polyéther.
La cire de silicone peut être présente à raison de 0-8% en poids dans la composition finale, de préférence à raison de 2-6%.

Les corps gras non siliconés utilisables dans la composition selon l'invention peuvent être des huiles ou mélanges d'huiles non siliconées, telles que des huiles végétales, animales, minérales, synthétiques ou des triglycérides d'acides gras.
On peut citer l'huile de paraffine, de vaseline, le perhydrosqualène, l'huile d'arara, l'huile d'amande douce, de calophyllum, de palme, de ricin, d'avocat, de jojoba, d'olive ou de germes de céréales.
On peut également utiliser des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools, les triglycérides caprylique/caprique, les triglycérides d'acides gras en C₁₀ à C₁₈.
On peut encore utiliser des huiles hydrogénées concrètes à 25°C telles que les huiles de ricin, de palme ou de coco hydrogénées, ou le suif hydrogéné; des mono-, di- tri- ou sucro- glycérides; des lanolines; des esters gras concrets à 25°C.

On peut également utiliser des cires non siliconées, parmi lesquelles on peut citer les cires animales telles que la cire d'abeilles; les cires végétales telles que la cire de Carnauba, de Candellila, d'Ourrury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Toutefois, on a constaté que l'invention permettait également de préparer une composition cosmétiquement acceptable ne comprenant, dans sa phase grasse, que des huiles de silicone cycliques et volatiles.
Ces huiles s'évaporant au contact de la peau, on peut ainsi obtenir des compositions cosmétiques ne présentant aucun effet gras, particulièrement appréciées des peaux à tendance grasse.

La composition comprend également des pigments habituellement utilisées dans de telles compositions cosmétiques.
Les pigments sont présents dans la phase grasse à raison de 1,5 à 12 % en poids de la composition finale.
Ils peuvent être blancs ou colorés, minéraux et/ou organiques et/ou nacrés.
On peut citer, sans effets limitatifs, le dioxyde de titane (TiO₂), l'oxyde de zinc (ZnO), le dioxyde de zirconium (ZrO₂), les oxydes de fer noir, jaune, rouge, brun, le dioxyde de cérium (CeO₂), l'oxyde de chrome, le bleu ferrique, parmi les pigments minéraux.
Parmi les pigments organiques, on peut citer le noir de carbone, et les laques de baryum, strontium, calcium, aluminium.
Parmi les pigments nacrés, on peut citer le mica recouvert d'oxyde de titane ou d'oxychlorure de bismuth ainsi que le mica titane coloré.
La composition peut également comprendre des charges.
Les charges, qui peuvent être présentes à raison de 0-12% de la composition finale peuvent être minérales ou de synthèse, lamellaires ou non lamellaires.
On peut citer le talc, le mica, la silice, le kaolin, les poudres de Nylon et de polyéthylène, le Téflon, l'amidon, le micatitane, la nacre naturelle, le nitrure de bore, les microsphères creuses telles que l'Expancel de Nobel Industrie, et les microbilles de résine de silicone (Tospearls par exemple).

Selon que la composition finale se présente sous forme d'une émulsion, d'une pâte anhydre ou non, d'une poudre compactée ou coulée ou sous toute autre forme cosmétique envisageable, elle peut comprendre les constituants habituellement utilisés.
Ces constituants sont de préférence choisis en fonction de l'effet cosmétique souhaité pour la composition finale, tel que la couvrance, la transparence, la matité et/ou l'aspect satiné.
On peut citer, sans effets limitatifs :
. les gélifiants comme les argiles modifiées connues sous les noms de bentone, vendues par la société NL Industrie et utilisées telles qu'elles ou préalablement conditionnées dans un gel; la silice hydrophobe; les cires, par exemple de polyéthylène; les sels gras d'aluminium; la carboxyméthylcellulose. Le pourcentage de gélifiant dans la composition sera choisi selon que l'on désire une formule souple ou crémeuse.
. les vitamines comme les tocophérols et leurs dérivés, la vitamine A et ses dérivés, la vitamines C et ses dérivés comme les esters gras dont le palmitate.
. les filtres solaires comme l'octylméthoxycinnamate (Parsol MCX), la 3-benzophénone (Uvinul M40), le butylméthoxydibenzoyl-méthane (Parsol 1789).
. les matériaux huileux comme les huiles végétales, les esters de synthèse, la lécithine, les parfums, les huiles essentielles.
. les agents hydratants, tels que le propylène glycol et le glycérol.

Lorsque la composition se présente sous-forme d'une émulsion, elle peut également comprendre un tensioactif, par exemple un tensioactif usuel anionique ou non ionique. Le tensioactif est de préférence présent, dans la phase aqueuse, à raison de 2-8% en poids de la composition.

Les procédés de fabrication des compositions selon l'invention ne différent en rien des procédés classiquement utilisés en cosmétique et parfaitement connus de l'homme de l'art.

Les compositions selon l'invention peuvent se présenter sous forme d'un produit de maquillage de la peau tel qu'un fond de teint, une crème teintée ou blanche, un rouge à lèvres, un mascara, une poudre libre de maquillage, un fard à joues ou à paupières.

L'invention est illustrée plus en détail dans les exemples suivants.

### Exemple 1

On prépare un fond de teint ayant la composition suivante:

### phase grasse

. cyclopentadiméthylsiloxane (huile D5) 9 g
. cyclohexadiméthylsiloxane (huile D6) 7,5 g
. cire de silicone polyéther 4 g
. huiles de silicone non volatiles
   (polyphénylméthylsiloxane et polycétylméthylsiloxane) 12 g
. mélange de gomme et d'huile de silicone
   (polydiméthyldiphénylsiloxane à 15% dans le cyclopentadiméthylsiloxane) 1 g
. émulsionnant 7,5 g
. pigments 11 g
. charges (poudre de Nylon, billes de résine siliconées) 3 g

### phase aqueuse

. sulfate de magnésium 1 g
. glycérol 2 g
. propylène glycol 3 g
. polyéthylène glycol 4,7 g
. carboxyméthylcellulose 0,1 g
. eau qsp 100 g

On prépare la composition en chauffant les constituants de la phase grasse, hormis les huiles volatiles, à 65°C et en mélangeant. On ajoute ensuite, à 60°C, les huiles volatiles. Parallèlement, on prépare la phase aqueuse en portant tous ses constituants à 80°C et en mélangeant. On laisse refroidir jusqu'à 30°C.
On mélange alors les deux phases à l'aide d'une turbine à une vitesse d'environ 2500 tr/min.
On obtient ainsi un fond de teint fluide, coloré et de texture agréable, qui s'étale bien et s'applique uniformément.
Le maquillage obtenu est uniforme et naturel, et conserve une bonne tenue.

### Exemple 2

On mesure la quantité d'huile de silicone volatile qui s'évapore au cours du temps, pour une composition maintenue à 25°C ou 32°C.
La température de 32°C étant une température très voisine de celle de la peau, on se rapproche ainsi des conditions lors d'une application cutanée.
Un échantillon de 3 mg de la composition préparée à l'exemple 1 est répartie de manière uniforme sur une plaque de verre de 3 cm² qui est placée dans un four à la température adéquate.
On détermine la quantité d'huile de silicone évaporée au cours du temps; on obtient les résultats suivants:

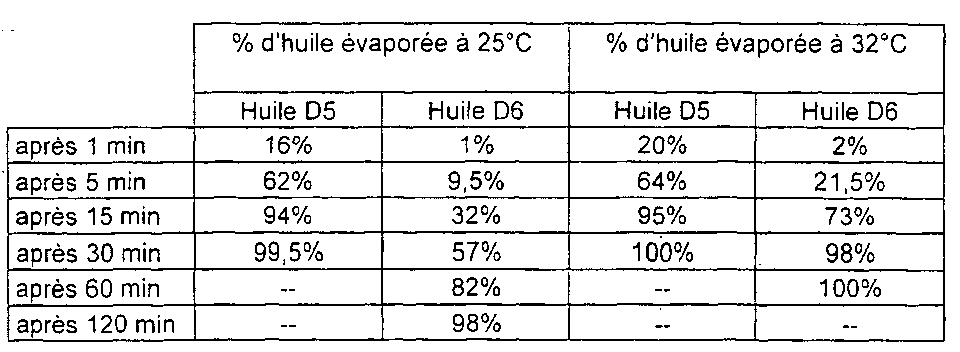

Le temps d'application moyen d'un fond de teint est d'environ 2 minutes.
On peut donc valablement estimer que, lors de l'application d'un fond de teint selon l'invention, la majeure partie de l'huile D5 (les 2/3) va être évaporée au bout de 5 minutes, l'huile D6 restant sur la peau pendant environ 30 à 60 minutes.

### Exemple 3

On compare, d'une manière qualitative, la composition de l'exemple 1 avec deux compositions témoins:
. la composition A comprend comme seule huile volatile de l'huile D5,
. la composition B comprend comme seule huile volatile de l'huile D6.
Le reste des constituants des compositions est identique.

On obtient les résultats suivants:
. la composition A est moins confortable à l'utilisation que la composition selon l'invention; elle est plus sèche et moins onctueuse à l'étalement.
. la composition B est plus présente à l'étalement que la composition selon l'invention; elle est également moins évanescente.

### Exemple 4

On prépare la composition de fond de teint suivante:

### phase grasse

. cyclopentadiméthylsiloxane 4 g
. cyclohexadiméthylsiloxane 10 g
. émulsionnant 3 g
. pigments 7 g
. charges 10 g

### phase aqueuse

. propylène glycol 6,5 g
. polyéthylène glycol 10 g
. eau qsp 100 g

On obtient ainsi un fond de teint qui s'applique facilement, et qui a comme particularité de ne contenir que des huiles volatiles.
Ainsi, après évaporation de ces deux huiles, on obtient un maquillage exempt de corps gras.

## Revendications

1. Composition cosmétique comprenant dans une phase grasse, l'association d'au moins deux huiles siliconées volatiles et cycliques, la cyclopentadiméthylsiloxane et la cyclohexadiméthylsiloxane, la composition comprenant en outre des pigments à raison de 1,5 à 12% en poids par rapport au poids de la composition.

2. Composition selon la revendication 1, dans laquelle la cyclopentadiméthylsiloxane est présente en une quantité de 2-18% en poids, et la cyclohexadiméthylsiloxane est présente en une quantité de 2-18% en poids, par rapport au poids total de la composition

3. Composition selon l'une des revendications précédentes, comprenant en outre d'autres huiles volatiles notamment choisies parmi les huiles de silicone cycliques ou linéaires, telles que la cyclotétradiméthylsiloxane, et/ou les huiles organiques notamment de paraffine.

4. Composition selon l'une des revendications précédentes, dans laquelle les huiles volatiles sont présentes à raison de 4-20% en poids par rapport au poids total de la composition.

5. Composition selon l'une des revendications précédentes comprenant en outre une huile de silicone non volatile, choisie parmi
. les polydiméthylsiloxanes de viscosité inférieure à 100 mPa.s,
. les alkyldiméthicones répondant à la formule: 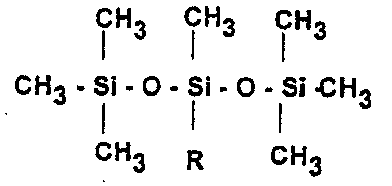 dans laquelle R représente le radical CₙH₂ₙ₊₁, avec n = 1 à 8.
. les polyphénylméthylsiloxanes.

6. Composition selon l'une des revendications précédentes, comprenant en outre une gomme de silicone, de préférence en une quantité de 0-1% en poids, notamment choisie parmi
. les gommes de formule : 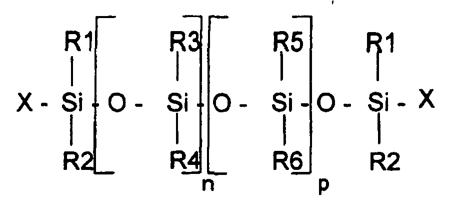
dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

7. Composition selon la revendication 6, dans laquelle la gomme de silicone est choisie parmi celles pour lesquelles :
. les substituants R1 à R6 et X représentent un groupement méthyle, p = 0 et n = 2700,
. les substituants R1 à R6 et X représentent un groupement méthyle, p = 0 et n = 2300,
. les substituants R1 à R6 représentent un groupement méthyle, le substituant X représente un groupement hydroxyle, p = 0 et n = 2700, en solution à 13 % dans du cyclopentasiloxane ou du polydiméthylsiloxane,
. les substituants R1, R2, R5, R6 et X représentent un groupement méthyle, les substituants R3 et R4 représentent un groupement aryle tel que le poids moléculaire du composé soit de 600 000.

8. Composition selon l'une des revendications précédentes, comprenant en outre une cire de silicone, de préférence à raison de 0-8% en poids.

9. Composition selon l'une des revendications précédentes, comprenant en outre un corps gras non siliconé choisi parmi
. les huiles végétales, animales, minérales, synthétiques ou des triglycérides d'acides gras, telles que l'huile de paraffine, de vaseline, le perhydrosqualène, l'huile d'arara, l'huile d'amande douce, de calophyllum, de palme, de ricin, d'avocat, de jojoba, d'olive ou de germes de céréales; les esters d'acide lanolique, d'acide oléique, d'acide laurique, d'acide stéarique ou d'acide myristique; les alcools tels que l'alcool oléique, l'alcool linoléique ou linolénique, l'alcool isostéarique ou l'octyl dodécanol; les acétylglycérides, les octanoates, décanoates ou ricinoléates d'alcools ou de polyalcools, les triglycérides caprylique/caprique, les triglycérides d'acides gras en C₁₀ à C₁₈; les huiles hydrogénées concrètes à 25°C telles que les huiles de ricin, de palme ou de coco hydrogénées, ou le suif hydrogéné; les mono-, di-, tri- ou sucro- glycérides; les lanolines; les esters gras concrets à 25°C;
et/ou
. les cires animales telles que la cire d'abeilles; les cires végétales telles que la cire de Carnauba, de Candellila, d'Ourrury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

10. Composition selon l'une des revendications précédentes, comprenant en outre des charges.

11. Composition selon l'une des revendications 1 à 4, dans laquelle la phase grasse ne comprend que des huiles de silicone cycliques et volatiles.

12. Composition selon l'une des revendications précédentes, se présentant sous forme d'un produit de maquillage de la peau tel qu'un fond de teint, une crème teintée ou blanche, un rouge à lèvres, un mascara, une poudre libre de maquillage, un fard à joues ou à paupières.

13. Utilisation dans une composition de maquillage comprenant une phase grasse, de l'association d'au moins deux huiles siliconées volatiles et cycliques, la cyclopentadiméthylsiloxane et la cyclohexadiméthylsiloxane, et de pigments à raison de 1,5 à 12% en poids par rapport au poids de la composition.

## Claims

1. Cosmetic composition comprising, in a fatty phase, a combination of at least two volatile and cyclic silicone oils, cyclopentadimethylsiloxane and cyclohexadimethylsiloxane, the composition also comprising pigments in a proportion of 1.5 to 12% by weight relative to the weight of the composition.

2. Composition according to Claim 1, in which the cyclopentadimethylsiloxane is present in an amount of 2-18% by weight and the cyclohexadimethylsiloxane is present in an amount of 2-18% by weight, relative to the total weight of the composition.

3. Composition according to either one of the preceding claims, also comprising other volatile oils chosen in particular from linear or cyclic silicone oils, such as cyclotetradimethylsiloxane, and/or organic oils, in particular paraffin oils.

4. Composition according to one of the preceding claims, in which the volatile oils are present in a proportion of 4-20% by weight relative to the total weight of the composition.

5. Composition according to one of the preceding claims, also comprising a non-volatile silicone oil, chosen from
• polydimethylsiloxanes with a viscosity of less than 100 mPa.s
• alkyldimethicones corresponding to the formula: 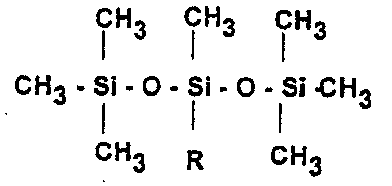 in which R represents the radical CₙH₂₊₁, with n = 1 to 8,
• polyphenylmethylsiloxanes.

6. Composition according to one of the preceding claims, also comprising a silicone gum, preferably in an amount of 0-1% by weight, chosen in particular from
- gums of formula: 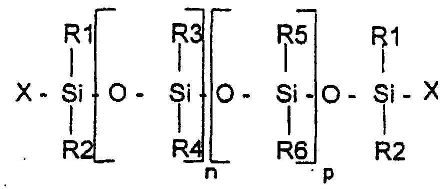 in which:
R₁, R₂, R₅ and R₆ are, together or separately, an alkyl radical having 1 to 6 carbon atoms,
R₃ and R₄ are, together or separately, an alkyl radical having from 1 to 6 carbon atoms or an aryl radical,
X is an alkyl radical having from 1 to 6 carbon atoms, a hydroxyl radical or a vinyl radical,
n and p being chosen so as to give the silicone gum a viscosity of greater than 100,000 mPa.s, preferably greater than 500,000 mPa.s.

7. Composition according to Claim 6, in which the silicone gum is chosen from those for which:
- the substituents R1 to R6 and X represent a methyl group, p=0 and n=2700.
- the substituents R1 to R6 and X represent a methyl group, p=0 and n=2300.
- the substituents R1 to R6 represent a methyl group, the substituent X represents a hydroxyl group, p=0 and n=2700, as a 13% solution in cyclopentasiloxane or polydimethylsiloxane,
- the substituents R1, R2, R5, R6 and X represent a methyl group, the substituents R3 and R4 represent an aryl group such that the molecular weight of the compound is 600,000.

8. Composition according to one of the preceding claims, also comprising a silicone wax, preferably in a proportion of 0-8% by weight.

9. Composition according to one of the preceding claims, also comprising a non-silicone fatty substance chosen from
- plant, animal, mineral or synthetic oils or fatty acid triglycerides, such as liquid paraffin, liquid petroleum jelly, perhydrosqualene, arara oil, sweet almond oil, beauty-leaf oil, palm oil, castor oil, avocado oil, jojoba oil, olive oil or cereal germ oil; esters of lanolic acid, of oleic acid, of lauric acid, of stearic acid or of myristic acid; alcohols such as oleyl alcohol, linoleyl alcohol or linolenyl alcohol, isostearyl alcohol or octyldodecanol; acetylglycerides, octanoates, decanoates or ricinoleates of alcohols or of polyalcohols, caprylic/capric triglycerides, triglycerides of C₁₀ to C₁₈ fatty acids, hydrogenated oils that are solid at 25°C, such as hydrogenated castor oil, palm oil or coconut oil, or hydrogenated tallow; mono-, di-, tri- or sucroglycerides; lanolins; fatty esters that are solid at 25°C;
and/or
- animal waxes such as beeswax; plant waxes such as carnauba wax, candelilla wax, ouricurry wax, Japan wax, cork fibre wax or sugar cane wax; mineral waxes, for example paraffin wax, lignite wax or microcrystalline waxes or ozokerites; synthetic waxes, including polyethylene waxes and the waxes obtained by Fischer-Tropsch synthesis.

10. Composition according to one of the preceding claims, also comprising fillers.

11. Composition according to one of Claims 1 to 4, in which the fatty phase comprises only volatile and cyclic silicone oils.

12. Composition according to one of the preceding claims, in the form of a make-up product for the skin, such as a foundation, a tinted or white cream, a lipstick, a mascara, a stand-alone make-up powder, a blusher or an eyeshadow.

13. Use, in a cosmetic composition comprising a fatty phase, of a combination of at least two volatile and cyclic silicone oils, cyclopentadimethylsiloxane and cyclohexadimethylsiloxane, and of pigments in a proportion of 1.5 to 12% by weight relative to the weight of the composition.

## Patentansprüche

1. Kosmetische Zusammensetzung, die in einer Fettphase die Kombination von mindestens zwei flüchtigen und cyclischen Siliconölen enthält, das Cyclopentadimethylsiloxan und das Cyclohexadimethylsiloxan, wobei die Zusammensetzung ferner Pigmente in Mengenanteilen von 1,5 bis 12 Gew.-%, bezogen auf das Gewicht der Zusammensetzung, enthält.

2. Zusammensetzung nach Anspruch 1, wobei das Cyclopentadimethylsiloxan in einer Menge von 2 bis 18 Gew.-% und das Cyclohexadimethylsiloxan in einer Menge von 2 bis 18 Gew.-%, bezogen auf das Gesamtgewicht der Zusammensetzung, vorliegt.

3. Zusammensetzung nach einem der vorhergehenden Ansprüche, die ferner andere flüchtige Öle enthält, die insbesondere unter den cyclischen oder geradkettigen Siliconölen, wie Cycloteträdimethylsiloxan, und/oder organischen Ölen, insbesondere Paraffin, ausgewählt sind.

4. Zusammensetzung nach einem der vorhergehenden Ansprüche, wobei die flüchtigen Öle in einem Mengenanteil von 4 bis 20 Gew.-%, bezogen auf das Gesamtgewicht der Zusammensetzung, vorliegen.

5. Zusammensetzung nach einem der vorhergehenden Ansprüche, die ferner ein nicht flüchtiges Siliconöl enthält, das ausgewählt ist unter:
- Polydimethylsiloxanen mit einer Viskosität unter 100 mPa·s;
- Alkyldimethiconen der folgenden Formel: 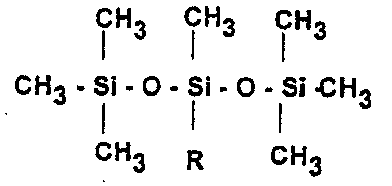 worin R eine Gruppe CₙH₂ₙ₊₁ mit n = 1 bis 8 bedeutet; und
- Polyphenylmethylsiloxanen.

6. Zusammensetzung nach einem der vorhergehenden Ansprüche, die ferner vorzugsweise in einer Menge von 0 bis 1 Gew.-% einen Silicongummi enthält, der insbesondere ausgewählt ist unter:
- den Gummis der folgenden Formel: 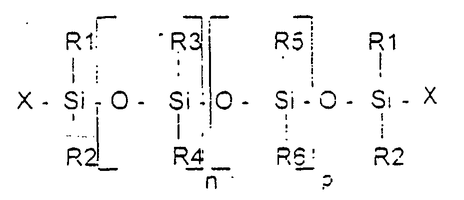
worin bedeuten:
R₁, R₂, R₅ und R₆ gleichzeitig oder unabhängig voneinander eine Alkylgruppe mit 1 bis 6 Kohlenstoffatomen,
R₃ und R₄ gleichzeitig oder unabhängig voneinander eine Alkylgruppe mit 1 bis 6 Kohlenstoffatomen oder eine Arylgruppe,
X eine Alkylgruppe mit 1 bis 6 Kohlenstoffatomen, eine Hydroxygruppe oder eine Vinylgruppe,
wobei n und p so ausgewählt sind, daß der Silicongummi eine Viskosität über 100 000 mPa·s und vorzugsweise über 500 000 mPa·s hat.

7. Zusammensetzung nach Anspruch 6, wobei der Silicongummi unter den Gummis ausgewählt ist, worin:
- die Substituenten R1 bis R6 und X Methyl bedeuten, p = 0 und n = 2700,
- die Substituenten R1 bis R6 und X Methyl bedeuten, p = 0 und n = 2300,
- die Substituenten R1 bis R6 Methyl bedeuten, der Substituent X eine Hydroxygruppe ist, p = 0 und n = 2700, 13%ige Lösung in Cyclopentasiloxan oder Polydimethylsiloxan,
- die Substituenten R1, R2, R5, R6 und X Methyl bedeuten und die Substituenten R3 und R4 eine Arylgruppe bedeuten, wobei das Molekulargewicht der Verbindung 600 000 beträgt.

8. Zusammensetzung nach einem der vorhergehenden Ansprüche, die ferner vorzugsweise in einer Menge von 0 bis 8 Gew.-% ein Siliconwachs enthält.

9. Zusammensetzung nach einem der vorhergehenden Ansprüche, die ferner eine nicht siliconhaltige Fettsubstanz enthält, die ausgewählt ist unter:
- pflanzlichen Ölen, tierischen Ölen, Mineralölen oder synthetischen Ölen oder Triglyceriden von Fettsäuren, wie Paraffinöl, Vaselineöl, Perhydrosqualen, Araraöl, Süßmandelöl, Calophyllumöl, Palmöl, Ricinusöl, Avocadoöl, Jojobaöl, Olivenöl oder Getreidekeimöl; Estern von Lanolinsäure, Ölsäure, Laurinsäure, Stearinsäure oder Myristinsäure; Alkoholen, wie Oleylalkohol, Linoleylalkohol oder Linolenylalkohol, Stearylalkohol oder Octyldodecanol; Acetylglyceriden, Octanoaten, Decanoaten oder Ricinoleaten von Alkoholen oder Polyalkoholen; Capryl/caprin-Triglyceriden; Triglyceriden von Fettsäuren mit 10 bis 18 Kohlenstoffatomen; bei 25 °C festen hydrierten Ölen, wie hydriertes Ricinusöl, Palmöl oder Cocosöl oder hydrierter Talg ; Mono-, Di-, Tri- oder Zuckerglyceriden; Lanolinen; und bei 25 °C festen Fettsäureestern;
und/oder
- tierischen Wachsen wie Bienenwachs; pflanzlichen Wachsen, wie beispielsweise Carnaubawachs, Candelillawachs, Ouricurywachs, Japanwachs oder Wachsen von Korkfasern oder Zuckerrohr; Mineralwachsen, beispielsweise Paraffinwachs, Lignit oder mikrokristallinen Wachsen oder Ozokeriten; synthetischen Wachsen, darunter Polyethylenwachsen und durch Fischer-Tropsch-Synthese hergestellten Wachsen.

10. Zusammensetzung nach einem der vorhergehenden Ansprüche, die ferner Füllstoffe enthält.

11. Zusammensetzung nach einem der Ansprüche 1 bis 4, wobei die Fettphase nur cyclische und flüchtige Öle enthält.

12. Zusammensetzung nach einem der vorhergehenden Ansprüche, die in Form eines Produktes zum Schminken der Haut beispielsweise als Make-up, weiße oder farbige Creme, Lippenstift, Mascara, loses Pulver zum Schminken, Wangenrouge oder Lidschatten vorliegt.

13. Verwendung der Kombination von mindestens zwei flüchtigen und cyclischen Siliconölen, des Cyclopentadimethylsiloxans und des Cyclohexadimethylsiloxans, und Pigmenten in Mengenanteilen von 1,5 bis 12 Gew.-%, bezogen auf das Gewicht der Zusammensetzung, in einer Zusammensetzung zum Schminken, die eine Fettphase enthält.
